# FASCICULE DE BREVET EUROPEEN

(11) **EP 1 574 214 B1**
(45) Date de publication et mention de la délivrance du brevet: **04.10.2006**
(21) Numéro de dépôt: 05006751.1
(22) Date de dépôt: 07.07.2000
(51) Int. Cl.: A61K 31/216, A61K 9/16

(54) **Composition pharmaceutique comprenant du fénofibrate et son procédé de préparation**
Pharmazeutische Zusammensetzung enthaltend Fenofibrat und Verfahren zu deren Herstellung
Pharmaceutical composition comprising fenofibrate and process for its preparation

(30) Priorité: 09.07.1999 FR 9908923
(43) Date de publication de la demande: 14.09.2005
(62) Demande divisionnaire de: 00949677.9
(73) Titulaire: ETHYPHARM, 78550 Houdan (FR)
(72) Inventeur: Criere, Bruno, 27930 Gravigny (FR); Chenevier, Philippe, Montreal Quebec H3T 1F7 (CA); Suplie, Pascal, 27400 Montaure (FR)
(74) Mandataire: Ahner, Francis

(56) Documents cités:
- WO-A-98/31361

## Description

La présente invention a pour objet une nouvelle composition pharmaceutique contenant du fénofibrate.

Le fénofibrate est préconisé dans le traitement des hyperlipidémies, des hypercholestérolémies et des hypertriglycéridémies endogènes de l'adulte. Un traitement de 300 à 400 mg de fénofibrate par jour permet une réduction de 20 à 25 % de la cholestérolémie et de 40 à 50 % de la triglycéridémie.

Le métabolite majeur plasmatique du fénofibrate est l'acide fénofibrique. La demi-vie plasmatique d'élimination de l'acide fénofibrique est de l'ordre de 20 heures. Sa concentration plasmatique maximale est atteinte en moyenne cinq heures après l'ingestion du médicament. La concentration plasmatique moyenne est de l'ordre de 15 microgrammes/ml pour une posologie de 300 mg de fénofibrate par jour. Ce taux est stable tout au long du traitement.

Le fénofibrate est un principe actif très faiblement soluble dans l'eau dont l'absorption au niveau du tractus digestif est limitée. Une augmentation de sa solubilité ou de sa vitesse de solubilisation entraîne une meilleure absorption digestive.

Diverses voies ont été explorées pour augmenter la vitesse de solubilisation du fénofibrate : la micronisation du principe actif, l'ajout d'un tensioactif, et la co-micronisation du fénofibrate avec un tensioactif.

Le brevet EP 256 933 décrit des granules de fénofibrate dans lesquels le fénofibrate est micronisé pour augmenter sa biodisponibilité. Les microparticules cristallines de fénofibrate sont de dimension inférieure à 50 µm. Le liant utilisé est la polyvinylpyrrolidone. Le document suggère d'autres types de liants comme les polymères métacryliques, les dérivés de cellulose, et les polyéthylènes glycols. Les granules décrits dans les exemples de EP 256 933 sont obtenus par un procédé mettant en oeuvre des solvants organiques.

Le brevet EP 330 532 propose d'améliorer la biodisponibilité du fénofibrate en le co-micronisant avec un tensioactif, comme le laurylsulfate de sodium. Le co-micronisat est ensuite granulé par voie humide afin d'améliorer les capacités d'écoulement de la poudre et de faciliter la mise en gélules. Cette co-micronisation permet une augmentation significative de la biodisponibilité par rapport à l'utilisation de fénofibrate décrite dans EP 256 933. Les granulés décrits dans EP 330 532 contiennent de la polyvinylpyrrolidone comme liant.

Ce brevet enseigne que la co-micronisation du fénofibrate avec un tensioactif solide améliore significativement la biodisponibilité du fénofibrate comparativement à l'utilisation d'un tensioactif, d'une micronisation ou de l'association d'un tensioactif et du fénofibrate micronisé.

Le brevet WO 98/31361 propose d'améliorer la biodisponibilité du fénofibrate, en fixant sur un support inerte hydrodispersible du fénofibrate micronisé, un polymère hydrophile et éventuellement un tensioactif. Le polymère hydrophile, identifié comme de la polyvinylpyrrolidone, représente au moins 20 % en poids de la composition précédemment décrite.

Ce procédé permet d'augmenter la vitesse de dissolution du fénofibrate, ainsi que sa biodisponibilité. Cependant, le procédé de préparation selon ce brevet n'est pas totalement satisfaisant, car il nécessite la mise en oeuvre d'une quantité importante de PVP et des autres excipients. L'exemple présenté dans cette demande de brevet, fait part d'une composition contenant seulement 17,7 % de fénofibrate exprimé en rapport massique. Ce faible rapport massique du fénofibrate entraîne une forme finale de très grande taille d'où une administration non aisée de la dose souhaitée de fénofibrate, ou l'administration de deux comprimés.

Il a été découvert dans le cadre de la présente invention que l'incorporation d'un dérivé cellulosique utilisé comme liant et adjuvant de solubilisation dans une composition contenant du fénofibrate micronisé et un tensioactif permet d'obtenir une biodisponibilité supérieure à une composition contenant un co-micronisat de fénofibrate et de tensioactif.

La présente invention a donc pour objet une composition pharmaceutique contenant du fénofibrate micronisé, un tensioactif de l'hydroxypropylméthylcellulose en tant que liant et adjuvant de solubilisation, caractérisée en ce que le rapport massique fénofibrate/hydroxypropylméthylcellulose est compris entre 5/1 et 15/1.

La composition de l'invention est avantageusement présentée en gélules contenant de la poudre ou des granules, de préférence sous la forme de granules. Ces granules peuvent notamment être préparés par montage sur des microgranules neutres, par pulvérisation d'une suspension aqueuse contenant le tensioactif, l'hydroxypropylméthylcellulose liant solubilisé et le fénofibrate micronisé en suspension, ou par granulation de poudre par voie humide selon laquelle les constituants dont notamment le fénofibrate micronisé, le tensioactif et le dérivé cellulosique sont granulés par granulation humide en utilisant une solution de mouillage aqueuse, séchés et calibrés.

La composition pharmaceutique selon la présente invention présente une forte proportion de fénofibrate, elle peut donc se présenter sous une formulation de taille inférieure aux formulations de l'art antérieur, ce qui rend cette composition selon l'invention, facilement administrable.

La quantité de fénofibrate est supérieure ou égale à 60 % en poids, de préférence supérieure ou égale à 70 % en poids, de préférence encore supérieure ou égale à 75 % en poids, par rapport au poids de la composition.

Dans le cadre de la présente invention, le fénofibrate n'est pas co-micronisé avec un tensioactif. Au contraire il est micronisé seul puis associé à un tensioactif et au dérivé cellulosique liant, adjuvant de solubilisation.

Le tensioactif est choisi parmi les tensioactifs solides ou liquides à température ambiante, par exemple le laurylsulfate de sodium, le Polysorbate® 80 ou le Montane® 20, de préférence le laurylsulfate de sodium.

L'agent tensioactif représente entre 1 à 10 %, de préférence entre 3 et 5 % en poids par rapport au poids de fénofibrate.

Le dérivé cellulosique liant représente entre 2 et 15 %, de préférence entre 5 et 12 % en poids de la composition.

On choisit de préférence l'hydroxypropylméthylcellulose dont la viscosité apparente est comprise entre 2,4 et 18 mPa.s (cP) et de manière encore plus préférée comprise entre 2,4 et 3,6 mPa.s (cP), comme par exemple le Pharmacoat 603®.

La taille moyenne des particules de fénofibrate est inférieure à 15 µm, de préférence 10 µm, de préférence encore inférieure à 8 µm.

La composition de l'invention peut en outre contenir au moins un excipient tel que les diluants comme le lactose, des agents anti-mousse comme le Diméthicone® et le Siméthicone®, des lubrifiants comme le talc.

La composition pharmaceutique de l'invention est avantageusement constituée de granules en une quantité équivalant à une dose de fénofibrate comprise entre 50 et 300 mg, de préférence égale à 200 mg.

La présente invention concerne également un procédé de préparation de la poudre ou des granules dont la composition est décrite précédemment. Ce procédé ne met en oeuvre aucun solvant organique.

Selon une première variante, les granules sont préparés par montage sur des microgranules neutres.

Les microgranules neutres ont une granulométrie comprise entre 200 et 1000 microns, de préférence entre 400 et 600 microns.

Le montage est effectué en turbine à dragéification, en turbine perforée ou en lit d'air fluidisé, de préférence en lit d'air fluidisé.

Le montage sur des microgranules neutres se fait par pulvérisation d'une suspension aqueuse contenant le tensioactif, l'hydroxypropylméthylcellulose liant solubilisé, et le fénofibrate micronisé en suspension.

Selon une deuxième variante, les granules sont obtenus par granulation de poudre par voie humide. La granulation permet de densifier les poudres et d'améliorer leurs propriétés d'écoulement. Elle permet également une meilleure conservation de l'homogénéïté, en évitant le démélange des différents constituants.

Le fénofibrate micronisé, le tensioactif, l'hydroxypropylméthylcellulose et éventuellement les autres excipients sont mélangés, granulés, séchés puis calibrés. La solution de mouillage peut être de l'eau ou une solution aqueuse contenant le dérivé cellulosique liant et/ou le tensioactif.

Selon un mode de mise en oeuvre particulier, le fénofibrate et les autres excipients sont mélangés dans un mélangeur planétaire. La solution de mouillage est amenée directement dans le mélange. La masse humidifiée obtenue est granulée avec un granulateur oscillant, puis séchée à l'étuve. Les granules sont obtenus après passage sur calibreur oscillant.
La figure 1 représente le profil de libération in vivo de la formulation de l'exemple 1C et d'une formulation de l'art antérieur chez des sujets à jeun.
La figure 2 représente le profil de libération in vivo de la formulation de l'exemple 1C et d'une formulation de l'art antérieur chez des sujets venant de s'alimenter.
La figure 3 représente le profil de libération in vivo de la formulation de l'exemple 2B et d'une formulation de l'art antérieur chez des sujets à jeun.
La figure 4 représente le profil de libération in vivo de la formulation de l'exemple comparatif 3 et d'une formulation de l'art antérieur chez des sujets venant de s'alimenter.

L'invention est illustrée par les exemples suivants.

### Exemple 1 : Granules

### 1A) Microgranules (XFEN 1735)

Les microgranules sont obtenus par pulvérisation d'une suspension aqueuse sur des noyaux neutres. La composition est présentée dans le tableau suivant :

| **Formule** | **Quantité (Pourcentage Massique)** |
|---|---|
| Fénofibrate micronisé | 64,5 |
| Microgranules neutres | 21 |
| HPMC (Pharmacoat 603®) | 11,2 |
| Polysorbate® 80 | 3,3 |
| Teneur en fénofibrate | 645 mg/g |

La dissolution in vitro est déterminée selon une méthode en cellule à flux continu avec un débit de 8 ml/min de laurylsulfate de sodium à 0,1 N. Les pourcentages de produit dissous en fonction du temps en comparaison avec une formulation de l'art antérieur, Lipanthyl 200 M, sont présentés dans le tableau suivant.

| | | |
|---|---|---|
| Temps (min) | 15 | 30 |
| Exemple 1 A (% dissous) | 73 | 95 |
| Lipanthyl 200M (% dissous) | 47,3 | 64,7 |

La formulation 1A présente une dissolution plus rapide que celle du Lipanthyl 200M.

### 1B) Microgranules (X FEN 1935)

La taille moyenne des particules de fénofibrate est égale à 6,9 ± 0,7 microns.

Les microgranules sont obtenus par pulvérisation d'une suspension aqueuse sur des noyaux neutres. La suspension contient du fénofibrate micronisé, du laurylsulfate de sodium et de l'HPMC.

Le montage est effectué en lit d'air fluidisé Huttlin (rotoprocess).

La formule obtenue est présentée ci-dessous.

| **FORMULE** | **QUANTITE (pourcentage massique)** |
|---|---|
| Fénofibrate micronisé | 65,2 |
| Microgranules neutres | 20,1 |
| HPMC (Pharmacoat 603®) | 11,4 |
| Laurylsulfate de sodium | 3,3 |
| Teneur en fénofibrate | 652 mg/g |

La taille des microgranules neutres est comprise entre 400 et 600 µm.

### 1C) Gélules de microgranules (Y FEN 001)

On prépare des microgranules de composition suivante :

| **MATIERES PREMIERES** | **QUANTITE (pourcentage massique)** |
|---|---|
| Fénofibrate micronisé | 67,1 |
| Microgranules neutres | 17,2 |
| Pharmacoat 603® (HPMC) | 11,7 |
| Laurylsulfate de sodium | 3,3 |
| Emulsion diméthicone 35 % | 0,2 |
| Talc | 0,5 |
| Teneur en fénofibrate | 671 mg/g |

### Selon le procédé décrit au paragraphe 1A).

Les microgranules obtenus sont répartis en gélules de taille 1, contenant chacune 200 mg de fénofibrate.

La dissolution in vitro est déterminée selon une méthode en cellule à flux continu avec un débit de 8ml/min de laurylsulfate de sodium à 0,1 N. Les résultats comparatifs avec une formulation de l'art antérieur, Lipanthyl 200M, sont présentés dans le tableau suivant.

| | | |
|---|---|---|
| Temps (min) | 15 | 30 |
| Exemple 1C (% dissous) | 76 | 100 |
| Lipanthyl 200M (% dissous) | 47,3 | 64,7 |

La formulation 1C présente une dissolution plus rapide que celle du Lipanthyl 200M.

Les gélules sont conservées pendant 6 mois à 40°C/75 % humidité relative. Les granules sont stables dans ces conditions de stockage accélérées. Les essais de dissolution in vitro (en cellules à flux continu avec un débit de 8 ml/min de laurylsulfate de sodium à 0,1 N) ont été effectués. Les pourcentages de produit dissous en fonction du temps pour des gélules conservées 1, 3 et 6 mois sont présentés dans le tableau suivant.

| **Temps de dissolution (min)** | **Temps de conservation** | | |
|---|---|---|---|
| | **1 mois (% produit dissous)** | **3 mois (% produit dissous)** | **6 mois (% produit dissous)** |
| 5 | 25,1 | 23,0 | 20,1 |
| 15 | 71,8 | 65,6 | 66,5 |
| 25 | 95,7 | 88,7 | 91,0 |
| 35 | 104,7 | 98,7 | 98,2 |
| 45 | 106,4 | 100,2 | 99,1 |
| 55 | 106,7 | 100,5 | 99,5 |
| 65 | 106,8 | 100,6 | 99,7 |

L'évolution de la teneur en principe actif au cours du stockage est présentée dans le tableau suivant.

| **Teneur (mg/gélule)** | **Temps de conservation** | | | |
|---|---|---|---|---|
| | **0** | **1 mois** | **3 mois** | **6 mois** |
| | 208,6 | 192,6 | 190,8 | 211,7 |

### Etude pharmacocinétique réalisée chez le sujet à jeun

On compare le profil de libération in vivo des gélules contenant les granules YFEN 01 dosées à 200 mg de fénofibrate, à celui des gélules commercialisées sous la marque Lipanthyl 200M.

Cette étude est réalisée chez 9 sujets. Des prélèvements sanguins sont réalisés à des intervalles de temps réguliers et on dose l'acide fénofibrique.

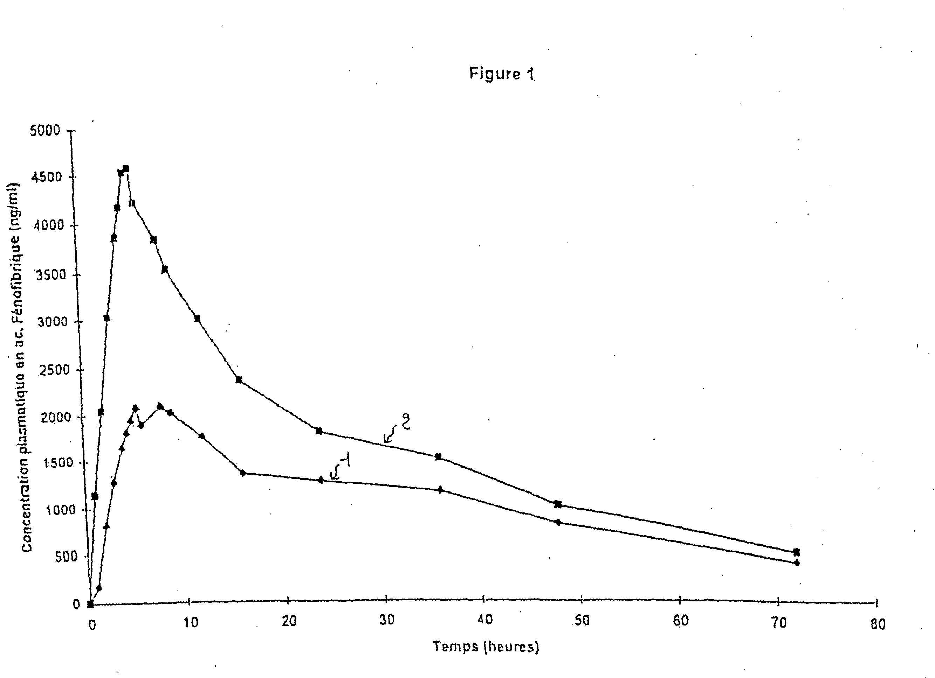

Les résultats sont présentés dans le tableau suivant et figure 1.

| **Paramètres pharmacocinétiques** | **Lipanthyl 200 M** | **Exemple 1C** |
|---|---|---|
| AUC₀₋ₜ | 76 | 119 |
| (µg.h/ml) | | |
| | | |
| AUC_{inf} | 96 | 137 |
| (µg.h/ml) | | |
| | | |
| Cₘₐₓ | 2,35 | 4,71 |
| (µg/ml) | | |
| | | |
| Tₘₐₓ | 8,0 | 5,5 |
| (heures) | | |
| | | |
| Ke | 0,032 | 0,028 |
| (1/heure) | | |
| | | |
| Elim ½ | 26,7 | 24,9 |
| (heures) | | |
| Les abréviations suivantes sont utilisées dans la présente demande : | | |
| Cₘₐₓ : concentration plasmatique maximale, | | |
| Tₘₐₓ : temps nécessaire pour atteindre le Cmax, | | |
| Elim_{1/2} : demi-vie plasmatique, | | |
| AUC₀₋ₜ : aire sous la courbe de 0 à t, | | |
| AUC_{0-∞} : aire sous la courbe de 0 à l'∞, | | |
| Ke : constante d'élimination. | | |

Les résultats obtenus pour le Lipanthyl 200 M et pour le produit de l'exemple 1C sont représentés sur la figure 1 respectivement par les courbes 1 et 2.

Ces résultats montrent que la composition suivant la présente invention a une biodisponibilité supérieure à celle du Lipanthyl 200 M chez le sujet à jeun.

### Etude pharmacocinétique réalisée chez le sujet venant de s'alimenter

On compare le profil de libération in vivo des gélules contenant les granules YFEN 01 dosées à 200 mg de fénofibrate, à celui des gélules commercialisées sous la marque Lipanthyl 200 M.

Cette étude est réalisée chez 18 sujets. Des prélèvements sanguins sont réalisés à des intervalles de temps réguliers et on dose l'acide fénofibrique.

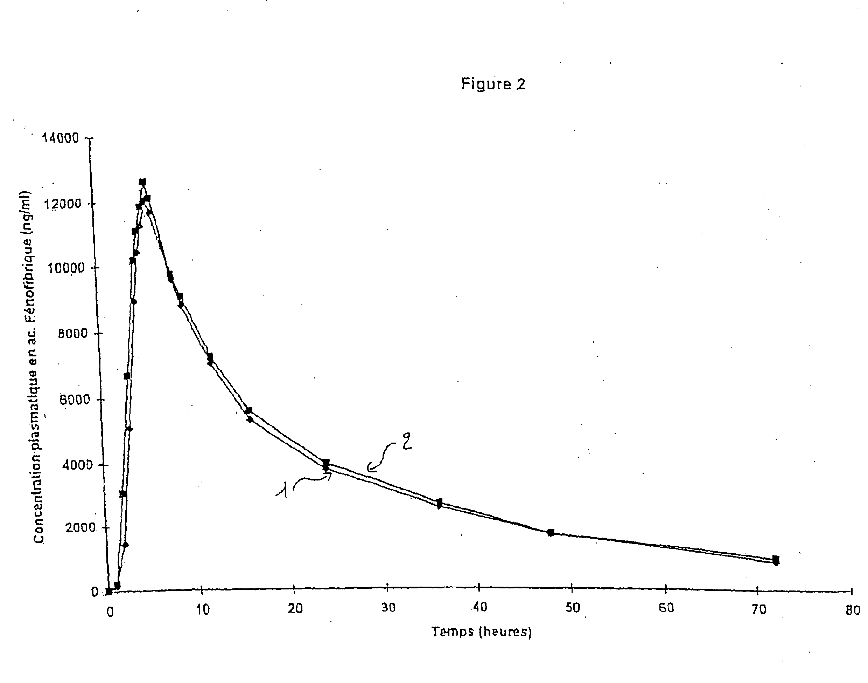

Les résultats sont présentés dans le tableau suivant et figure 2.

| **Paramètres pharmacocinétiques** | **Lipanthyl 200 M** | **Exemple 1C** |
|---|---|---|
| AUC₀₋ₜ | 244 | 257 |
| (µg.h/ml) | | |
| | | |
| AUC_{inf} | 255 | 270 |
| (µg.h/ml) | | |
| | | |
| Cₘₐₓ | 12 | 13 |
| (µg/ml) | | |
| | | |
| Tₘₐₓ | 5,5 | 5,5 |
| (heures) | | |
| | | |
| Ke | 0,04 | 0,04 |
| (1/heure) | | |
| | | |
| Elim ½ | 19,6 | 19,3 |
| (heures) | | |

Les résultats obtenus pour le Lipanthyl 200 M et pour le produit de l'exemple 1C sont représentés sur la figure 2 respectivement par les courbes 1 et 2.

Ces résultats montrent que la composition suivant la présente invention est bioéquivalente à celle du Lipanthyl 200M chez le sujet qui vient de s'alimenter.

### Exemple 2 : Poudre

### 2A) Granulés (X FEN 1992)

On prépare des granulés de composition suivante

| **FORMULE** | **POURCENTAGE MASSIQUE** |
|---|---|
| Fénofibrate micronisé | 71 |
| Lactose | 21,5 |
| HPMC (Pharmacoat 603®) | 5 |
| Laurylsulfate de sodium | 2,5 |

Le fénofibrate micronisé, l'HPMC et le lactose sont mélangés à l'aide d'un mélangeur planétaire. Ce mélange est granulé en présence d'une solution de laurylsulfate de sodium.

Le temps d'écoulement des granulés est de 7s. L'aptitude au tassement et la répartition granulométrique sont présentées dans les tableaux suivants. Ces mesures ont été effectuées conformément aux normes de la Pharmacopée Européenne.

| **Aptitude au tassement (X FEN 1992)** | |
|---|---|
| V0 | 204 ml |
| V10 | 186 ml |
| V500 | 168 ml |
| V1250 | 164 ml |
| V10-V500 | 22 ml |

| **Répartition granulométrique (X FEN 1992)** | |
|---|---|
| **Ouverture de maille des tamis (mm)** | **% de masse de refus** |
| 0,6 | 8 |
| 0,5 | 9 |
| 0,355 | 12 |
| 0,2 | 30 |
| 0,1 | 23 |
| 0 | 18 |

### 2B) Gélules de granulés (Y FEN 002)

### • Préparation

Le fénofibrate micronisé est mélangé dans un mélangeur PMA (Niro Fielder) avec du lactose et de l'HPMC, puis mouillé avec une solution aqueuse de laurylsulfate de sodium. La masse obtenue est granulée par passage sur un granulateur oscillant, séchée puis calibrée sur un tamis de 1,25 mm d'ouverture de maille.

Les granulés sont ensuite conditionnés en gélules, de taille 1 dosées à 200 mg de fénofibrate.

On obtient des granulés de composition suivante.

| **FORMULE** | **POURCENTAGE MASSIQUE** |
|---|---|
| Fénofibrate micronisé | 70 |
| Lactose | 21,5 |
| Pharmacoat 603® (HPMC) | 5 |
| Laurylsulfate de sodium | 3,5 |
| Teneur | 700 mg/g |

### • Propriétés des granulés

Le temps d'écoulement des granulés est de 6 s. L'aptitude au tassement et la répartition granulométrique sont présentées dans les tableaux suivants. Ces mesures ont été effectuées conformément aux normes de la Pharmacopée Européenne.

| **Aptitude au tassement (Y FEN 002)** | |
|---|---|
| V0 | 216 ml |
| V10 | 200 ml |
| V500 | 172 ml |
| V1250 | 170 ml |
| V10-V500 | 28 ml |

| **Répartition granulométrique (Y FEN 002)** | |
|---|---|
| **Ouverture de maille des tamis (mm)** | **% de masse de refus** |
| 0,6 | 5 |
| 0,5 | 7 |
| 0,355 | 11 |
| 0,2 | 30 |
| 0,1 | 25 |
| 0 | 22 |

La dissolution in vitro est déterminée selon une méthode en cellule à flux continu avec un débit de 8 ml/min de laurylsulfate de sodium à 0,1 N. Les résultats comparatifs avec une formulation de l'art antérieur, Lipanthyl 200 M, sont présentés dans le tableau suivant.

| | | |
|---|---|---|
| Temps (min) | 15 | 30 |
| Exemple 2B (% dissous) | 82,2 | 88,5 |
| Lipanthyl 200 M (% dissous) | 47,3 | 64,7 |

La formulation 2B présente une dissolution plus rapide que celle du Lipanthyl 200 M.

### • Essais de stabilité

Les gélules conservées à 40°C / 75 % d'humidité relative sont stables pendant 6 mois.

Les essais de dissolution in vitro (en cellules à flux continu avec un débit de 8 ml/min de laurylsulfate de sodium à 0,1N) ont été effectués. Les pourcentages de produit dissous en fonction du temps pour des gélules conservées 1, 3 et 6 mois sont présentés dans le tableau suivant.

| **Temps de dissolution (min)** | **Temps de conservation** | | |
|---|---|---|---|
| | **1 mois (% produit dissous)** | **3 mois (% produit dissous)** | **6 mois (% produit dissous)** |
| 5 | 54,2 | 52,9 | 49,0 |
| 15 | 81,1 | 75,8 | 82,2 |
| 25 | 86,4 | 79,6 | 87,2 |
| 35 | 88,8 | 81,6 | 89,8 |
| 45 | 90,7 | 82,9 | 91,5 |
| 55 | 92,1 | 83,9 | 92,7 |
| 65 | 93,2 | 84,7 | 93,6 |

L'évolution de la teneur en principe actif au cours du stockage est présentée dans le tableau suivant.

| | | | | |
|---|---|---|---|---|
| **Teneur (mg/gélule)** | **Temps de conservation** | | | |
| | **0** | **1 mois** | **3 mois** | **6 mois** |
| | 196,6 | 190,0 | 199,8 | 203,3 |

### Etude pharmacocinétique réalisée chez le sujet à jeun

On compare le profil de libération in vivo des gélules contenant les granules YFEN 002 dosées à 200 mg de fénofibrate, à celui des gélules commercialisées sous la marque Lipanthyl 200 M.

Cette étude est réalisée chez 9 sujets. Des prélèvements sanguins sont réalisés à des intervalles de temps réguliers et on dose l'acide fénofibrique.

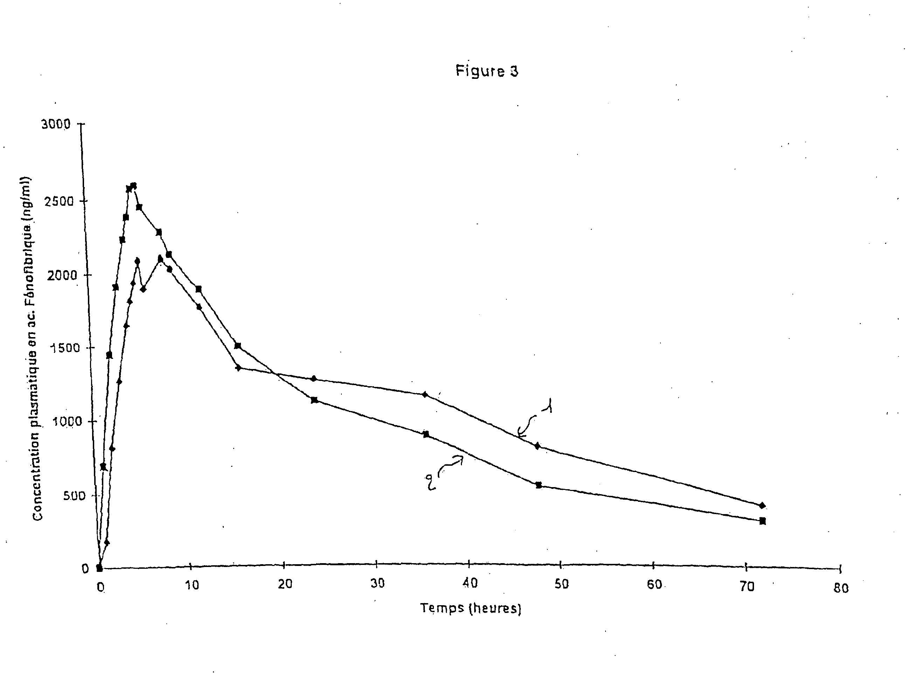

Les résultats sont présentés dans le tableau suivant et figure 3.

| **Paramètres pharmacocinétiques** | **Lipanthyl 200 M** | **Exemple 2B** |
|---|---|---|
| AUC₀₋ₜ | 76 | 70 |
| (µg.h/ml) | | |
| | | |
| AUC_{inf} | 96 | 82 |
| (µg.h/ml) | | |
| | | |
| Cₘₐₓ | 2,35 | 2,8 |
| (µg/ml) | | |
| | | |
| Tₘₐₓ | 8,0 | 5,5 |
| (heures) | | |
| | | |
| Ke | 0 ,032 | 0,033 |
| (1/heure) | | |
| | | |
| Elim ½ | 26,7 | 23,1 |
| (heures) | | |

Les résultats obtenus pour le Lipanthyl 200 M et pour le produit de l'exemple 2B sont représentés sur la figure 3 respectivement par les courbes 1 et 2.

Ces résultats montrent que la composition de l'exemple 2B est bioéquivalente à celle du Lipanthyl 200 M chez le sujet à jeun.

### Exemple 3 comparatif : lot ZEF 001

Cet exemple illustre l'art antérieur.

Il associe la micronisation du fénofibrate et l'utilisation d'un tensioactif. Il se différencie de la présente invention par l'utilisation d'un mélange d'excipients liants constitué d'un dérivé cellulosique, autre que l'HPMC : l'Avicel PH 101 et de polyvinylpyrrolidone (PVP K30).

Il est préparé par extrusion-sphéronisation.

### • Formule théorique

| **Produits** | **Quantité théorique (%)** |
|---|---|
| Fénofibrate micronisé | 75,08 |
| Montanox 80® | 4,72 |
| Avicel PH 101® | 5,02 |
| PVP K 30® | 4,12 |
| Explotab® | 11,06 |

### • Profil de dissolution in vitro

La dissolution in vitro est déterminée selon une méthode en cellule à flux continu avec un débit de 8 ml/min de laurylsulfate de sodium à 0,1 N. Les résultats comparatifs avec le Lipanthyl 200 M, sont présentés dans le tableau suivant.

| | | |
|---|---|---|
| Temps (min) | 15 | 30 |
| Exemple 3 (% dissous) | 24 | 40 |
| Lipanthyl 200 M (% dissous) | 47,3 | 64,7 |

La dissolution est plus lente que celle observée pour le Lipanthyl 200 M.

### Etude pharmacocinétique réalisée chez le sujet à jeun

On compare le profil de libération in vivo des gélules contenant les granules ZEF 001 dosées à 200 mg de fénofibrate, à celui des gélules commercialisées sous la marque Lipanthyl 200 M.

Cette étude est réalisée chez 5 sujets à jeun, recevant une dose unique. Des prélèvements sanguins sont réalisés à des intervalles de temps réguliers et on dose l'acide fénofibrique.

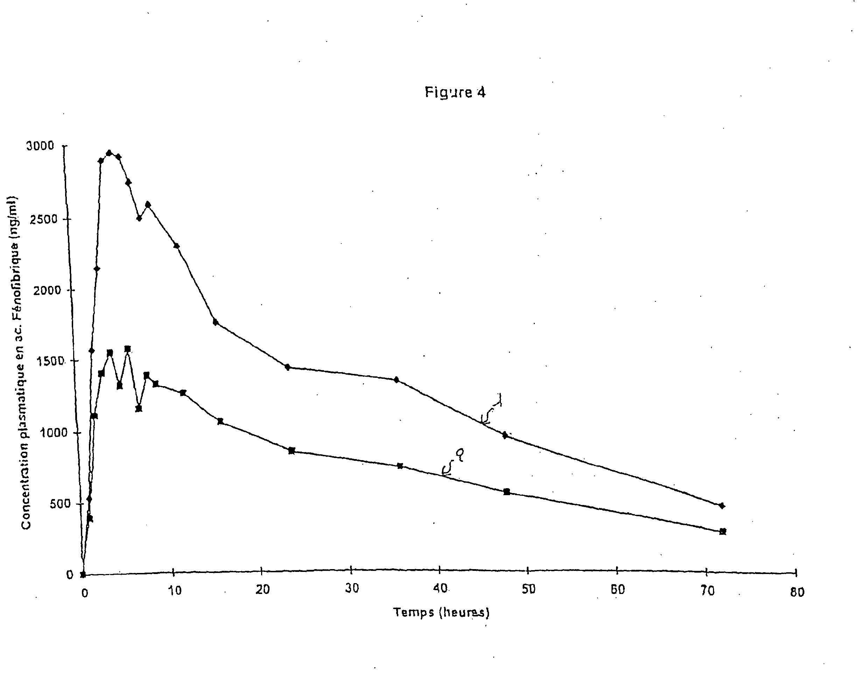

Les résultats sont présentés dans le tableau suivant et figure 4.

| **Paramètres pharmacocinétiques** | **Lipanthyl 200 M** | **Exemple 3** |
|---|---|---|
| AUC₀₋ₜ | 92 | 47 |
| (µg.h/ml) | | |
| | | |
| AUC_{inf} | 104 | 53 |
| (µg.h/ml) | | |
| | | |
| Cₘₐₓ | 3,5 | 1,7 |
| (µg/ml) | | |
| | | |
| Tₘₐₓ | 5,6 | 4,6 |
| (heures) | | |
| | | |
| Ke | 0,04 | 0,038 |
| (1/heure) | | |
| | | |
| Elim ½ | 18,9 | 20,3 |
| (heures) | | |

Les résultats obtenus pour le Lipanthyl 200 M et pour le produit de l'exemple 3 sont représentés sur la figure 4 respectivement par les courbes 1 et 2.

Ces résultats montrent la biodisponibilité supérieure du Lipanthyl 200 M par rapport à cette formulation s'appuyant sur l'art antérieur.

L'exemple 3, montre que la combinaison des connaissances de l'art antérieur (à savoir micronisation ou utilisation de tensioactifs) ne permet pas d'obtenir une dissolution rapide du fénofibrate. Ceci se traduit par une faible biodisponibilité comparativement au Lipanthyl 200 M.

Les compositions réalisées suivant la présente invention montrent une dissolution plus rapide que la formule de l'art antérieur et une biodisponibilité améliorée.

## Revendications

1. Composition pharmaceutique contenant du fénofibrate micronisé, un tensioactif et de l'hydroxypropylméthylcellulose en tant que liant et adjuvant de solubilisation, **caractérisée en ce que** le rapport massique fénofibrate/(hydroxypropylméthylcellulose en tant que liant et adjuvant de solubilisation) est compris entre 5/1 et 15/1.

2. Composition selon la revendication 1, **caractérisée en ce que** l'hydroxypropylméthylcellulose a une viscosité apparente comprise entre 2,4 et 18 mP·as (cP) de préférence comprise entre 2,4 et 3,6 mPa·s (cP)

3. Composition selon la revendication 1 ou 2, **caractérisée en ce qu**'elle contient une quantité de fénofibrate supérieure ou égale à 60 % en poids, de préférence supérieure ou égale à 70 % en poids, de préférence encore supérieure ou égale à 75 % en poids, par rapport au poids de la composition.

4. Composition selon l'une quelconque des revendications précédentes, **caractérisée en ce que** le tensioactif est choisi dans le groupe formé par le polysorbate® 80, le Montane® 20 et le laurylsulfate de sodium.

5. Composition selon l'une quelconque des revendications précédentes, **caractérisée en ce que** le tensioactif représente entre 1 et 10 %, de préférence entre 3 et 5 % en poids par rapport au poids du fénofibrate.

6. Composition selon l'une quelconque des revendications précédentes, **caractérisée en ce que** l'hydroxypropylméthylcellulose en tant que liant et adjuvant de solubilisation représente entre 2 et 15 %, de préférence entre 5 et 12 % en poids de la composition.

7. Composition selon l'une quelconque des revendications précédentes, **caractérisée en ce qu**'elle contient au moins un excipient, tel qu'un diluant comme le lactose, un agent anti-mousse comme le Diméthicone® ou le Siméthicone®, ou un lubrifiant comme le talc.

8. Composition selon l'une quelconque des revendications précédentes, **caractérisée en ce que** la taille moyenne des particules de fénofibrate est inférieure à 15 µm, de préférence inférieure à 8 µm.

9. Composition selon l'une quelconque des revendications précédentes, **caractérisée en ce qu**'elle est sous la forme de poudre ou de granules, éventuellement contenus dans des gélules.

10. Procédé de préparation de la composition selon l'une quelconque des revendications précédentes, **caractérisé en ce que** des granules sont préparés par montage sur des microgranules neutres, par pulvérisation d'une suspension aqueuse contenant le tensioactif, l'hydroxypropylméthylcellulose solubilisé et le fénofibrate micronisé en suspension.

11. Procédé de préparation de la composition selon l'une quelconque des revendications 1 à 9, **caractérisé en ce que** des granules sont obtenus par granulation de poudre par voie humide selon laquelle les constituants dont notamment le fénofibrate micronisé, le tensioactif et l'hydroxypropylméthylcellulose sont granulés par granulation humide en utilisant une solution de mouillage aqueuse, séchés et calibrés.

## Claims

1. Pharmaceutical composition containing micronized fenofibrate, a surfactant and hydroxypropylmethylcellulose as binder and solubilization adjuvant, **characterized in that** the fenofibrate/(hydroxypropylmethylcellulose as binder and solubilization adjuvant) ratio by mass is between 5/1 and 15/1.

2. Composition according to Claim 1, **characterized in that** the hydroxypropylmethylcellulose has an apparent viscosity of between 2.4 and 18 mPa.s (cP), preferably of between 2.4 and 3.6 mPa.s (cP).

3. Composition according to Claim 1 or 2, **characterized in that** it contains an amount of fenofibrate of greater than or equal to 60% by weight, preferably greater than or equal to 70% by weight, even more preferably greater than or equal to 75% by weight, relative to the weight of the composition.

4. Composition according to any one of the preceding claims, **characterized in that** the surfactant is chosen from the group formed by polysorbate® 80, Montane® 20 and sodium lauryl sulphate.

5. Composition according to any one of the preceding claims, **characterized in that** the surfactant represents between 1% and 10%, preferably between 3% and 5% by weight, relative to the weight of the fenofibrate.

6. Composition according to any one of the preceding claims, **characterized in that** the hydroxypropylmethylcellulose as binder and solubilization adjuvant represents between 2% and 15%, preferably between 5% and 12% by weight of the composition.

7. Composition according to any one of the preceding claims, **characterized in that** it contains at least one excipient, such as a diluent, for instance lactose, an antifoam agent, for instance Dimethicone® or Simethicone®, or a lubricant, for instance talc.

8. Composition according to any one of the preceding claims, **characterized in that** the average size of the fenofibrate particles is less than 15 µm, preferably less than 8 µm.

9. Composition according to any one of the preceding claims, **characterized in that** it is in the form of powder or granules, optionally contained in gelatin capsules.

10. Process for preparing the composition according to any one of the preceding claims, **characterized in that** granules are prepared by coating on neutral microgranules, by spraying of an aqueous suspension containing the surfactant, the solubilized hydroxypropylmethylcellulose and the micronized fenofibrate in suspension.

11. Process for preparing the composition according to any one of Claims 1 to 9, **characterized in that** granules are obtained by wet granulation of powder, according to which the constituents, including in particular the micronized fenofibrate, the surfactant and the hydroxypropylmethylcellulose, are granulated by wet granulation using an aqueous wetting solution, dried and calibrated.

## Patentansprüche

1. Pharmazeutische Zusammensetzung, die mikronisiertes Fenofibrat, ein Tensid und Hydroxypropylmethylcellulose als Bindemittel und Löslichmachungs-Adjuvans enthält, **dadurch gekennzeichnet, dass** das Massenverhältnis Fenofibrat/(Hydroxypropylmethylcellulose als Bindemittel und als Löslichmachungs-Adjuvans) zwischen 5/1 und 15/1 einschließlich liegt.

2. Zusammensetzung nach Anspruch 1, **dadurch gekennzeichnet, dass** die Hydroxypropylmethylcellulose eine scheinbare Viskosität zwischen 2,4 und 18 mPa.s (cP), vorzugsweise zwischen 2,4 und 3,6 mPa.s (cP) aufweist.

3. Zusammensetzung nach Anspruch 1 oder 2, **dadurch gekennzeichnet, dass** sie eine Fenofibrat-Menge von mehr als oder gleich 60 Gew.-%, bevorzugt mehr als oder gleich 70 Gew.-%, noch mehr bevorzugt mehr als oder gleich 75 Gew.-%, bezogen auf das Gewicht der Zusammensetzung, aufweist.

4. Zusammensetzung nach irgendeinem der vorangehenden Ansprüche, **dadurch gekennzeichnet, dass** das Tensid ausgewählt ist aus der Gruppe bestehend aus Polysorbat® 80, Montane® 20 und Natriumlaurylsulfat.

5. Zusammensetzung nach irgendeinem der vorangehenden Ansprüche, **dadurch gekennzeichnet, dass** das Tensid zwischen 1 und 10 Gew.-%, bevorzugt zwischen 3 und 5 Gew.-%, bezogen auf das Gewicht von Fenofibrat, ausmacht.

6. Zusammensetzung nach irgendeinem der vorangehenden Ansprüche, **dadurch gekennzeichnet, dass** die Hydroxypropylmethylcellulose als Bindemittel und als Löslichmachungs-Adjuvans zwischen 2 und 15 Gew.-%, bevorzugt zwischen 5 und 12 Gew.-% der Zusammensetzung ausmacht.

7. Zusammensetzung nach irgendeinem der vorangehenden Ansprüche, **dadurch gekennzeichnet, dass** sie mindestens einen Hilfsstoff, wie ein Verdünnungsmittel, wie Lactose, ein Schaumverhütungsmittel, wie Dimethicon® oder Simethicon®, oder ein Gleitmittel, wie Talkum, enthält.

8. Zusammensetzung nach irgendeinem der vorangehenden Ansprüche, **dadurch gekennzeichnet, dass** die mittlere Größe der Fenofibrat-Teilchen unterhalb von 15 µm, vorzugsweise unterhalb von 8 µm liegt.

9. Zusammensetzung nach irgendeinem der vorangehenden Ansprüche, **dadurch gekennzeichnet, dass** sie in Form von Pulver oder Granalien vorliegt, gegebenenfalls in Kapseln enthalten.

10. Verfahren zur Herstellung der Zusammensetzung nach irgendeinem der vorangehenden Ansprüche, **dadurch gekennzeichnet, dass** die Granalien durch Aufbringen auf Mikrogranalien-Neutralteilchen mittels Zerstäuben einer wässrigen Suspension, die das Tensid, die gelöste Hydroxypropylmethylcellulose und das mikronisierte Fenofibrat in Suspension enthält, hergestellt werden.

11. Verfahren zur Herstellung der Zusammensetzung nach irgendeinem der Ansprüche 1 bis 9, **dadurch gekennzeichnet, dass** die Granalien durch Granulation von Pulver auf feuchtem Weg erhalten werden, gemäß welcher die Bestandteile, unter diesen insbesondere das mikronisierte Fenofibrat, das Tensid und die Hydroxypropylmethylcellulose, durch feuchte Granulation unter Verwendung einer wässrigen Befeuchtungslösung granuliert, getrocknet und klassiert werden.
